(19) Europäisches Patentamt
European Patent Office
Office européen des brevets

(11) EP 3 596 472 B1

(12) **EUROPEAN PATENT SPECIFICATION**

(45) Date of publication and mention of the grant of the patent:
**10.04.2024 Bulletin 2024/15**

(21) Application number: **18714623.8**

(22) Date of filing: **01.03.2018**

(51) International Patent Classification (IPC):
***G01N 33/66*** *(2006.01)*

(52) Cooperative Patent Classification (CPC):
**G01N 33/66**

(86) International application number:
**PCT/NO2018/050055**

(87) International publication number:
**WO 2018/169408 (20.09.2018 Gazette 2018/38)**

# (54) FLUID COMPOSITION, METHOD FOR PREPARING THE COMPOSITION AND USE

FLÜSSIGE ZUSAMMENSETZUNG, VERFAHREN ZUR HERSTELLUNG DER ZUSAMMENSETZUNG UND VERWENDUNG

COMPOSITION DE FLUIDE, PROCÉDÉ DE PRÉPARATION DE LA COMPOSITION ET SON UTILISATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.03.2017 NO 20170385**

(43) Date of publication of application:
**22.01.2020 Bulletin 2020/04**

(73) Proprietor: **Lifecare AS**
**5152 Bønes (NO)**

(72) Inventor: **FRISVOLD, Rune**
**5224 Nesttun (NO)**

(74) Representative: **Zacco Norway AS**
**P.O. Box 488 Skøyen**
**0213 Oslo (NO)**

(56) References cited:
**EP-A1- 2 055 226**
**WO-A1-2006/061207**
**WO-A2-2004/056311**

- **OLGA KRUSHINITSKAYA ET AL: "The assessment of potentially interfering metabolites and dietary components in blood using an osmotic glucose sensor based on the concanavalin Adextran affinity assay", BIOSENSORS AND BIOELECTRONICS, ELSEVIER BV, NL, vol. 28, no. 1, 9 July 2011 (2011-07-09), pages 195-203, XP028340772, ISSN: 0956-5663, DOI: 10.1016/J.BIOS.2011.07.019 [retrieved on 2011-07-20]**
- **ADAMS GARY G ET AL: "Rheological and diffusion properties of a dextran-con A polymer in the presence of Insulin and Magnesium", RHEOLOGICA ACTA, DIETRICH STEINKOPFF VERLAG, DARMSTADT, DE, vol. 45, no. 5, 8 November 2005 (2005-11-08), pages 611-620, XP036030668, ISSN: 0035-4511, DOI: 10.1007/S00397-005-0013-Y [retrieved on 2005-11-08]**
- **Ramljak S ET AL: "& Moderated Poster Discussion ADA-Supported Research CLINICAL THERAPEUTICS/NEW TECHNOLOGY-GLUCOSE MONITORING AND SENSING: In vitro proof of principle experiment with the osmotic pressurebased sencell implantable glucose sensor technology", , 10 June 2016 (2016-06-10), XP055473961, Retrieved from the Internet: URL:http://diabetes.diabetesjournals.org/content/diabetes/65/Supplement_1/A221.full. pdf [retrieved on 2018-05-09]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 596 472 B1

## Description

TECHNICAL FIELD

The present application relates to fluid compositions, in special active fluid compositions which may be used in glucose sensor systems, for example a glucose sensor which relies on osmotic pressure as sensing principle.

BACKGROUND

Commonly glucose measurement is based on manual point sample sensors instrumentation (finger-pricking). However, devices capable of conducting continuous blood glucose measurements would provide the most complete picture of the glucose variations during the course of the day and prevent the onset of dangerous events by for example trigger an alarm function when the blood glucose moves beyond what are considered safe levels. This is especially important when persons are sleeping or not being able to look after themselves. Although the continuous blood glucose measurement instrumentation is considered as the most effective method of monitoring glucose, the transcutaneous nature of the sensor patches, combined with limited sensor lifetimes and long start-up periods, has meant that the single use sensor for manual point sampling remains the most common.

There are some drawbacks to the manual point sampling method. The persons often experience pain and discomfort with the manual point sample devices, which might in turn compromise such self-testing regimes. Incomplete numbers of measurements taken during the course of a day may result in that the average person with diabetes spending periods during the days in a hyperglycaemic or hypoglycaemia state. Both these conditions are potentially dangerous and can contribute to vascular damage, mental confusion and even death.

The benefits of existing continuous sensing technologies come with major drawbacks and disadvantages, and hence there are currently no real commercial alternatives to the manual point sample method. Existing continuous glucose measurements technologies systems are inconvenient, complicated and costly. There are no alarm functions or digital memory for storing the data. The existing systems also have a limited operational lifetime and require frequent calibrations using external point sample meters.

Detecting glucose by the principle of osmotic pressure holds promise of a glucose sensing technology that is suitable for both miniaturisation and long term continuous monitoring *in vivo* without causing patient discomfort or reducing quality of life. An osmotic sensor for measuring blood glucose is described in the PhD Thesis "Osmotic sensor for blood glucose monitoring applications ", by Olga Krushinitskaya, Department of Micro- and Nanosystems Technology, Vestfold University College, August 2012. The project in this PhD work addressed the technological aspect of developing a novel glucose sensor that was capable of tracking glucose continuously through the recording of osmotic pressure, based on the principle of utilizing the diffusion of water down its own concentration gradient, which enables an inherently simple sensor design in which the generated pressure is a function of the glucose concentration.

The osmotic sensor developed in the said project was based on the osmotic pressure generated by the competitive bonding between the sugar binding lectin Concanavalin A (ConA) and the long chained polysaccharide dextran, which forms a large macromolecular complex. Lectins are a group of proteins that have special binding sites for carbohydrates, and the ConA attaches strongly to glucose. The studies in the above Thesis exploited the osmotic effect generated by the competitive bonding of ConA and dextran in the presence of glucose. As the concentration of glucose is increased, more of the larger ConA-dextran macromolecular complexes are split up into the smaller ConA-glucose and free dextran "sub units". In this manner the number of free particles inside the sensor is increased as a function of glucose, leading to a corresponding rise in the osmotic pressure, see Figure. 1A-B.

This process is reversible and as the glucose concentrations falls, the Con A reattaches back to the dextran forming a large macromolecular complex from the Con A and dextran "sub units". The corresponding decrease in the number of free particles triggers the osmotic pressure to fall.

Granted European patent EP 1 631 187 B1 discloses a sensor for *in vivo* measurement of osmotic changes. The sensor is an invasive sensor which can be implanted subcutaneously, and specially an invasive sensor comprising at least one differential pressure-transducer that measures the pressure difference between two fluid volumes confined by, in one end the at least one differential pressure-transducer, and in the other end osmotic membranes.

The sensor described in EP 1 631 187 B1 can be utilized to monitor any changes within the in chemistry *in vivo.* The type of solutes and their concentration observed *in vivo* gives a tremendous amount of information regarding the physiology of the body, and its condition. By measuring the composition for instance in the interstitial fluid (ISF), a lot of information can be obtained regarding de-hydration of the body and different diseases: diabetes, kidney functi, etc. Also normal variations for instance in lactate concentrations caused by physical activity can be monitored.

In addition to the substances mentioned above, which can change the osmolality in the body, one can also find substances which by medication give an osmotic contribution in the body fluid.

Measurement of glucose in ISF is becoming recognized as an alternative to measuring the glucose directly in the blood. The glucose measurement in blood

is associated with several drawbacks. It needs a sample of blood, drawn from the body. Even though the equipment has become more sensitive, and therefore requires less blood, the process is associated with pain and the number of tests typically limited to less than 10 per day. It is also known that large variations in measured values can be caused by the measurement procedure.

Embodiments and details of the sensor are shown in figures 1-6B in EP 1 631 187 B1 and in the description paragraphs [0022] to [0054].

Further documents disclosing composition for use as active fluids in a continuous glucose sensor; EP 2 055 226; WO 2006/061207; WO 20047056311; OLGA KRUSHINITSKAYA ET AL: "The assessment of potentially interfering metabolites and dietary components in blood using an osmotic glucose sensor based on the concanavalin Adextran affinity assay", BIOSENSORS AND BIOELECTRONICS, ELSEVIER BV, NL, vol. 28, no. 1, 9 July 2011, pages 195-203; ADAMS GARY GET AL: "Rheological and diffusion properties of a dextran-con A polymer in the presence of Insulin and Magnesium", RHEOLOGICA ACTA, DIETRICH STEINKOPFF VERLAG, DARMSTADT, DE, vol. 45, no. 5, 8 November 2005, pages 611-620, XP036030668,ISSN: 0035-4511, DOI: 10.1007 S00397-005-0013-Y and Ramljak S ET AL: "& Moderated Poster Discussion ADA-Supported Research CLINICAL THERAPEUTICS/NEW TECHNOLOGY- GLUCOSE MONITORING AND SENSING: In vitro proof of principle experiment with the osmotic pressurebased sencell implantable glucose sensor technology", 10 June 2016, XP055473961.

The current invention, as defined in the appended claims, concerns a composition which can be used as an active fluid in continuous glucose sensing technology without the above described disadvantages. It was found that an optimal active fluid composition should have low viscosity and the viscosity should be substantially independent from the glucose concentration. The present compositions of the active fluids provide faster response time compared to previous known compositions due to lower fluid viscosity and optimal composition. The handling of the active fluids are also easier due to the lower viscosity. Further, the optimal compositions result in measurable osmotic pressure changes by the pressure sensor employed, i.e. the system sensitivity is highly improved. The active fluids chemistry exhibit reproducible concentrations, show longtime stable responses at room temperature (>3 months), and the concentrations are stable at 37 °C.

Thus, the active fluid composition according to the invention has advantageous effect on

- sensor lifetime; due to high fluid stability, no precipitation of the active compounds, and no leakage of active compounds through membrane;
- measurement response time; due to low fluid viscosity;
- measurement response asymmetry; due to viscosity is substantially independent from the glucose concentration,
- measurement sensitivity; due to optimal concentration and choice of compounds.

## SUMMARY OF THE INVENTION

In a first aspect the present invention provides an active fluid composition for use as an active fluid in a continuous glucose sensor comprising a lectin, the lectin being Concanavalin A (ConA); a lectin-binding molecule having a molecular weight of 10-100 kDa, the lectin-binding molecule being dextran; at least one chloride salt of a divalent metal ion chosen from $MgCl_2$, $CaCl_2$ and MnCh or a combination thereof, wherein the concentration of ConA is measuring the changes in the concentrations of glucose in fluids *in vitro* or *in vivo* by detecting osmotic pressure differences, and the glucose concentrations is tracjed continuously through the recording of osmotic pressure, for measuring glucose concentrations *in vitro* or *in vivo*

In a first embodiment the ConA to dextran molar ratio is 1:1.

In a second embodiment, the composition provided comprises dextran 40 kDa or dextran 70 kDa and an aqueous buffer solution with pH 7.4-7.5 containing Tris buffer 100 mM, 10 mM $MgCl_2$, 10 mM $CaCl_2$, 150 mM NaCl and 30 mM glucose.

In a second aspect the present invention provides a method for preparing a composition suitable for use as an active fluid in a continuous glucose sensor, according to any of the above embodiments, comprising the following steps

(i) preparing an aqueous buffer solution having pH from 7.0 to 7.8, comprising at least one chloride salt of a divalent metal chosen from $MgCl_2$, $CaCl_2$ and MnCh or a combination thereof, and optionally glucose,

(ii) either

(a) providing the desired amount of a lectin-binding molecule, the lectin-binding molecule being a polysaccharide, to which the aqueous buffer solution prepared in step (i) is added and left stirring until homogenized and dissolved, and thereafter providing the desired amount of a carbohydrate-binding molecule, the carbohydrate-binding molecule being a lectin to which the said buffer solution containing said lectin-binding molecule is added and left stirring until homogenized and dissolved, or

(b) providing the desired amount of a carbohydrate-binding molecule, the carbohydrate-binding molecule being a lectin, to which the aqueous buffer solution prepared in step (i) is added and left stirring until homogenized and dissolved, and thereafter providing the desired

amount of a lectin-binding molecule, the lectin-binding molecule being a polysaccharide, to which the said buffer solution containing said lectin is added and left stirring until homogenized and dissolved.

In a first embodiment of the method, the solutions in step (ii) is stirred for 2-48 hours, for instance 6-36 hours, or 12-24 hours for dissolution and homogenization of the said lectin and said polysaccharide in the aqueous buffer solution.

In any of the above embodiments of the method, the active fluid may be degassed in order to minimize formation of bubbles.

In a specific embodiment of the method, the fluid composition is prepared by the following steps

- preparing an aqueous buffer solution containing 100 mM Trizma buffer, 10 mM $MgCl_2$, 10 mM $CaCl_2$, 150 mM NaCl, and 30 mM glucose,
- verifying pH 7.4-7.5,
- the desired quantity of dextran is weighed and buffer solution is added and left overnight while stirring, giving a dissolved and homogenized dextran solution,
- the desired quantity of ConA is weighed out and dextran solution is added and left under stirring for 24 hours before use.

The aqueous buffer solution may be degassed in order to minimize formation of bubbles. After the degassing the volume should be checked and made up if necessary.

The composition according to present invention may be used as an active fluid in a sensor for measuring the changes in the concentrations of carbohydrates in fluids *in vitro* or *in vivo* by detecting osmotic pressure differences. In an embodiment the active fluid composition according to present invention may be used for measuring blood glucose concentrations *in vitro* or *in vivo*. The said composition may be used as an active fluid in an osmotic glucose sensor that is capable of tracking glucose concentrations continuously through the recording of osmotic pressure, for measuring glucose concentrations *in vitro* or *in vivo.*

In the context of present invention the terms "composition", "active fluid", "fluid composition", "reference fluid" are all expressions referring to the composition according to the present invention.

BRIEF DESCRIPTION OF THE DRAWINGS:

Figure 1: Illustration of sensing principle; Fig. 1A. Low glucose, low pressure difference; Fig. 1B. High glucose, high pressure difference. Fig 1C. Macrocell *in-vitro*; Fig. 1D. Linear correlation between osmotic pressures and glucose levels.

Figure 2: Viscosity as a function of glucose concentration for dextran 10kDa, 40kDa and 70kDa. ConA concentration is equal to 3mM, dextran concentration is 0.5mM. Please note the y-logarithmic scale.

Figure 3: Viscosity as a function of glucose concentration for dextran 10kDa, 40kDa and 70kDa. ConA concentration is equal to 1.5 mM, dextran concentration is 0.5 mM. Please note the y-logarithmic scale.

Figure 4: Viscosity as a function of glucose concentration for dextran 10kDa, 40kDa and 70kDa. ConA concentration is equal to 1 mM, dextran concentration is 1 mM. Please note the y-logarithmic scale.

Figure 5: Viscosity as a function of glucose concentration for two dextrans, 40kDa and 70k. ConA and dextran concentrations are equal to 1.5 mM.

Figure 6: Viscosity as a function of glucose concentration for dextran 40kDa, with two different ConA and dextran concentrations (1 and 1.5 mM).

Figure 7: Viscosity as a function of glucose concentration for dextran 70kDa, with two different ConA and dextran concentrations (1 and 1.5 mM).

Figure 8: An example curve showing changes between 2 mM glucose solution and 30 mM glucose solution. Note the strong fast spike followed by a slow drift in the opposite direction to reach a stable equilibrium.

DETAILED DESCRIBTION OF THE INVENTION

The object of present invention is to provide a composition which can be used as an active fluid in continuous glucose sensing technology. This object have been achieved by the composition comprising

a lectin, the lectin being Concanavalin A (ConA);
a lectin-binding molecule having a molecular weight of 10-100 kDa, the lectin-binding molecule being dextran;
at least one chloride salt of a divalent metal ion chosen from $MgCl_2$, $CaCl_2$ and MnCh or a combination thereof, wherein the concentration of ConA is measuring the changes in the concentrations of glucose in fluids *in vitro* or *in vivo* by detecting osmotic pressure differences, and glucose concentrations are

tracked continuously through the recording of osmotic pressure, for measuring glucose concentrations *in vitro* or *in vivo.*

The above composition comprises a carbohydrate-binding molecule which is a lectin. Lectins are carbohydrate-binding proteins, macromolecules that are highly specific for sugar moieties. Concanavalin A (ConA) is a lectin originally extracted from the jack-bean, *Canavalia ensiformis.* It is a member of the legume lectin family. It binds specifically to certain structures found in various sugars, glycoproteins, and glycolipids, mainly internal and nonreducing terminal α-D-mannosyl and α-D-glucosyl groups. It is known to exhibit long term chemical stability at physiological body temperatures. The configuration of ConA depends on the pH. Monomeric subunits are formed at pH 4-6 in the presence of 2-propanol, dimeric at pH 4.5-6.5, whereas the tetrameric structure is formed at a pH higher than 7. The size of the Con A monomer is approximately 42x40x39 Å. The molecular weight of one such sub-unit range from 25500 Da to 27000 Da, depending on the literature reference that is consulted. One sub-unit contains one binding site for certain structures found in sugars, e.g. glucose or mannose, and considering the tetrameric structure, such a molecule would have a total of 4 binding sites. The affinity towards carbohydrates is governed by a metal ion binding site that both activates Con A for saccharide binding as well as modulating its stability. $Ca^{2+}$, $Mg^{2+}$ and $Mn^{2+}$ may be used for activating the ConA, the $Mn^{2+}$ ion can be replaced by $Co^{2+}$, $Ni^{2+}$, $Zn^{2+}$ and $Cd^{2+}$.

Further, the above composition comprises a lectin-binding molecule. In present work dextran has been used as lection-binding molecule. Dextran is a complex branched glucan (polysaccharide made of many glucose molecules) composed of chains of varying lengths (from 3 to 2000 kDa. The straight chain consists of α-1,6 glycosidic linkages between glucose molecules, while branches begin from α-1,3 linkages. The branching of dextran can be from 0.5-60%, with the solubility decreasing as the branching is increased. Dextran is synthesized from sucrose by certain lactic acid bacteria, the best-known being *Leuconostoc mesenteroides* and *Streptococcus mutans.* The chemical formula for dextran is (from Mehvar et al. Dextran for trageted and sustained delivery of therapeutic and imaging agents. Journal of Controlled release, 2000. 69: p. 1-25.)

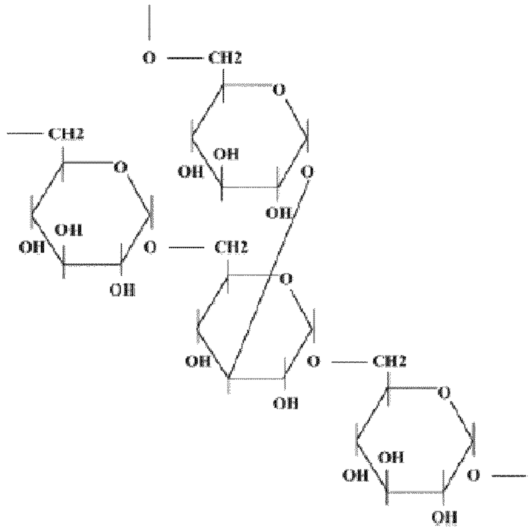

The above composition further includes at least one chloride salt of a divalent metal ion. The at least one chloride salt of a divalent metal ion is chosen from $MgCl_2$, $CaCl_2$ and $MnCl_2$. The at least one chloride salt, or a combination thereof is dissolved in an aqueous buffer solution giving concentrations 1-10 mM $MgCl_2$, 1-10 mM $CaCl_2$ and 1-10 mM MnCh. The aqueous solution may also comprise NaCl giving an isotonic solution.

In the composition according to present invention the buffer used for preparing the aqueous buffer solution may be chosen from

Tris buffer, other names tris(hydroxymethyl)aminomethane or THAM, is an organic compound with the formula (HOCH2)3CNH2. Tris buffer is also known as Trizma®, which is a trademark belonging to Sigma-Aldrich®. Tris is used as a component of buffer solutions, such as in TAE (Tris-acetate-EDTA) and TBE (Tris-barate-EDTA) buffer. TAE buffer is a buffer solution containing a mixture of Tris base, Acetic acid and EDTA. TBE buffer is a buffer solution containing a mixture of Tris base, Boric acid and EDTA. The Tris-buffered saline (TBS) is a buffer used in some biochemical techniques and is isotonic and non-toxic. TBS contains Tris and NaCl. The $pK_a$ of Tris buffers is dependent on temperature, the $pK_a$ declines approximately 0.03 units per degree Celsius rise in temperature.

or

HEPES buffer (4-(2-hydroxyethyl)-1-piperazineethanesulfonic acid; IUPAC name: 2-[4-(2-hydroxyethyl)piperazin-1-yl]ethanesulfonic acid) which is a zwitterionic organic chemical buffering agent. The buffer solution may be a combination of HEPES acid/Na HEPES salt and NaCl

In the composition according to the present invention glucose may further be added for the purpose of reducing the viscosity if the active fluid composition. At low glucose concentrations, dextran molecules are cross-linked by ConA bonds, forming an extremely viscous solution. When the glucose concentration increases, dextran molecules are partially replaced by glucose

at the binding sites of ConA. As a result, the network ConA-dextran is weakened and the viscosity of the active fluid decreases.

NaCl may be added to the active fluid composition to produce an isotonic solution.

The water used for preparing the active fluid, that is the buffer solution is a purified type of water such as reverse osmosis water, deionized or distilled water.

The sensing principle of an implantable glucose sensor relies on osmotic pressure variations between a reagent chamber and the solution, see Figures 1A-D. Detection of glucose is based on the competition between glucose and a polysaccharide (dextran) to bind to a receptor, the lectin Concanavalin A (ConA). ConA and dextran are present in the reagent chamber in an "active fluid" and are not to exit through the nanoporous membrane. At low glucose concentration, the pressure difference in the reagent chamber is low. Glucose molecules and small ions will pass through the pores of the membranes. Glucose molecules which enter the reagent chamber compete with dextran to bind to ConA proteins. As the concentration of glucose increases the release of dextran from ConA results in an increase of osmotic pressure that can be used to quantify the concentration of glucose. This process is reversible and as the glucose concentrations falls, the ConA reattaches back to the dextran forming a large macromolecular complex from the ConA and dextran "sub units". The corresponding decrease in the number of free particles triggers the osmotic pressure to fall.

The osmotic pressure $\Pi$ of an ideal solution of low concentration can be approximated using the Morse equation:

$$\pi = iMRT$$

where i is the dimensionless Van't Hoff factor, M is the molarity, R the gas constant and T the temperature of the chamber.

Figure 1D shows the linear correlation between osmotic pressures and glucose levels, accurate also at hypo and hyper glycemic levels.

The inventors found that lowering the viscosity of the composition would consequently reduce the asymmetry and response time of the sensor. In order to diminish the viscosity of the active fluid, the following parameters were explored while varying the glucose concentration in the solutions:

- Molecular weight of the dextran (10kDa, 40kDa or 70kDa)
- Ratio of ConA to dextran
- Initial concentration of ConA.

Figure 2 shows viscosity as a function of glucose concentration for dextran 10kDa, 40kDa and 70kDa, the molar ratio between ConA to dextran was 6:1, using initial concentrations respectively 3 mM and 0.5 mM. This active fluid composition was considered as a "baseline" for the viscosity measurements.

Lowering the ConA concentration and more specifically, the ConA to dextran concentrations ratio, was expected to decrease the number of intermolecular bonds between dextran and ConA, thus lowering the viscosity of the system. To investigate this effect, the ConA to dextran was reduced from 6:1 to 3:1, using the following concentrations ConA 1.5 mM, dextran 0.5 mM and glucose range 2 to 30 mM.

Figure 3 clearly shows that the viscosity of each system is decreased by one to two orders of magnitude when the ConA concentration is divided by two (all other parameters are kept constant). This is expected to have an extremely significant effect on the response time and the asymmetry of the response with increasing or decreasing glucose concentration.

Decreasing the molar ratio of ConA to dextran to 1:1 was shown to to improve the amplitude of the response of the sensor. Lowering the number of intermolecular bonds between ConA and dextran also lowered the viscosity. The inventors chose to test a slightly higher concentration of dextran (1 mM instead of 0.5 mM) since it was also shown to improve the amplitude of the response and the sensitivity of the sensor. An active fluid with a ConA and dextran concentrations of 1 mM showed an amplitude of response approximately three times larger than the amplitude of response of the "baseline" active fluid described above.

By keeping the molar concentrations of dextran and ConA equal (at 1 mM), the viscosity was further decreased by an order of magnitude when compared to 3:1 molar ratio of ConA to dextran, see Figure 4. Furthermore, the influence of the glucose concentration on viscosity is lessened. This decreased the asymmetry of the response times to ascending and descending glucose concentrations.

For characterisations, each solution was stirred during 24 hours before viscosity measurements. A Brookfield DV-II+Pro viscometer was used for all measurements. The viscometer drives a spindle through a calibrated spring which is immersed in the active fluid. The viscous drag of the fluid against the spindle is measured by the spring deflection, which is measured with a rotary transducer. The measurement range is determined by the rotational speed of the spindle, the size and shape of the spindle, the container where the spindle is rotating in, and the full scale torque of the calibrated spring. The viscosity appears in units of centipoise (shown "cP"). One centipoise is equal to 1 mPa.s in USI.

To control the temperature (T) at which the measurements were done, the viscometer was equipped with a water bath which can be set at the chosen T. All the viscosity measurements were performed at 37 °C, with a 5 min. waiting period to ensure stabilisation of the

temperature in the active fluid.

Based on viscosity measurements and simulation results, the inventors found clear trends in term of sensitivities and kinetics of the sensor can be established:

- A 1 to 1 molar concentration ratio of ConA to dextran was advantageous with respect to the sensitivities in term of osmotic pressure in the range of glucose concentration [0;30] mM.
- A 1 to 1 molar concentration ratio of ConA to dextran was advantageous with respect to fast kinetics and the least asymmetrical response to glucose variations since the viscosity of the active fluid varied very little with the glucose concentration.
- At 1 to 1 molar concentration ratio of ConA to dextran, a high ConA concentration (=dextran concentration) gave better sensitivity. However, it was also likely to increase the viscosity of the system, in turn increasing the time response of the sensor.
- Experimental studies of the viscosities of different active fluids were performed. These showed a rapid drop (of 3 orders of magnitude) as the concentration of ConA was decreased from 3 mM (based on monomer concentration) to 1 mM. Changes in the concentration of dextran also influenced the viscosity but to a lesser extent. In contrast the molecular weight of the dextran (10kDa, 40kDa, 70kDa) could change the viscosity by orders of magnitude, see figures 6 and 7.
- The lower the MW of the dextran, the lower the viscosity was. This means that an active fluid containing dextran 10kDa will give a faster response than one containing dextran 40kDa, which in turn is expected to give a faster response than one containing dextran 70kDa.
- Higher MW of the dextran, provided higher differential osmotic pressure of the system. Using a higher size dextran (e.g. 70kDa) in the active fluid is therefore going to improve the sensitivity of the system.
- Simulations of the response time based on these measurements indicated that number of active fluids are expected to have response times (in both increasing a decreasing glucose concentrations) of less than 5 minutes.

The inventors performed a number of experiments to test the response of different active fluids, e.g.: 1.0 mM ConA, 1.0 mM dextran 40 kDa and 1.0 mM ConA, 1.0 mM dextran 70 kDa. 1.5 mM ConA, 1.5 mM dextran 40 kDa and 1.5 mM ConA, 1.5 mM dextran 70 kDa.

<u>Test of active fluids based on 1.5 mM ConA, 1.5 mM dextran 40 kDa</u>

Experiments were carried out by cycling the contents of the sample chamber in a macrocell between 2 mM glucose (in a solution containing 100 mM Trizma B buffer, 150 mM NaCl, 10mM MgCh and 10mM $CaCl_2$) and 30 mM glucose in the same solution. At each change of glucose concentration, the solution was removed by hand using a pipette, taking great care not to touch the nanoporous (NP) membrane but bringing the pipette tip as close as possible to the bottom of the sample chamber. The solution was then replaced by the new chosen solution, which was agitated by "pumping" the pipette. The solution was removed and replaced by fresh solution another two times. Temperature was 21 °C.

Following this procedure, a reproducible signal was obtained. A change from 2 mM glucose to 30 mM glucose resulted in a large spike down in the measured pressure, followed by a slow rise to a new pressure that was approximately 10 mbar higher than the original value. A change from 30 mM glucose to 2 mM glucose gave a large spike up in measured pressure followed by a slow drift downwards (see figure 8). Both the fast spike in one direction and the slow drift in the opposite direction occurred each time.

These two features can be understood when we consider the time course of the events taking place in sample chamber and active fluid chamber, as described below.

1) Starting at low glucose concentration, to begin with, all small solutes are present in equal concentrations on both sides of the NP membrane. Most of the ConA and dextran inside the active fluid chamber is bound together, giving a small overpressure inside the chamber.

2) Glucose is added outside the NP membrane. The concentration of solutes is briefly higher outside the membrane than inside. There is a brief underpressure inside the chamber.

3) The glucose diffuses through the membrane. Small solutes are present in equal concentrations on both sides of the NP membrane. ConA and dextran dissociate and the measured pressure increases to finally give an overpressure inside the chamber.

Thus the observed spike is not an artifact, but shows the process of equilibration of the glucose concentration on the two sides of the membrane.

Results of the study on the active fluids also shows that the present active fluid compositions could be studied for 3 months with no perceptible loss of sensitivity, i.e. long term stability.

Having described preferred embodiments of the invention it will be apparent to those skilled in the art that other embodiments incorporating the concepts may be used. These and other examples of the invention illustrated above are intended by way of example only and the actual scope of the invention is to be determined from the following claims.

## Claims

1. Composition for use as an active fluid in a continuous glucose sensor comprising:

   a lectin, the lectin being Concanavalin A (ConA); a lectin-binding molecule having a molecular weight of 10-100 kDa, the lectin-binding molecule being dextran;
   at least one chloride salt of a divalent metal ion chosen from $MgCl_2$, $CaCl_2$ and $MnCl_2$ or a combination thereof,
   the composition being **characterized by**:
   the concentration of ConA is 1-1.5 mM based on the monomer concentration and the concentration of dextran is 1-1.5 mM wherein the continuous glucose sensor is :

   - measuring the changes in the concentrations of glucose in fluids *in vitro* or *in vivo* by detecting osmotic pressure differences, and
   - tracking glucose concentrations continuously through the recording of osmotic pressure, for measuring glucose concentrations *in vitro* or *in vivo*.

2. Composition according to claim 1, wherein the ConA to dextran molar ratio is 1:1.

3. A composition according to any of the proceeding claims, wherein the composition being **characterized by** :

   the dextran is dextran 40 kDa or dextran 70 kDa; an aqueous buffer solution with pH 7.4-7.5 containing Tris buffer 100 mM, 10 mM $MgCl_2$, 10 mM $CaCl_2$, 150 mM NaCl and 30 mM glucose.

4. Method for preparing a composition suitable for use as an active fluid in a continuous glucose sensor according to claims 1-3, comprising the following steps

   (i) preparing an aqueous buffer solution having pH from 7.0 to 7.8, comprising at least one chloride salt of a divalent metal chosen from $MgCl_2$, $CaCl_2$ and MnCh or a combination thereof, and optionally glucose,
   (ii) either:

   - (a) providing the desired amount of a lectin-binding molecule, the lectin binding molecule being dextran having a molecular weight of 10-100 kDa , to which the aqueous buffer solution prepared in **step (i)** is added and left stirring until homogenized and dissolved, and thereafter providing the desired amount of a lectin, the lectin being ConA to which the said buffer solution containing said dextran is added and left stirring until homogenized and dissolved, or
   - (b) providing the desired amount of a lectin, the lectin being ConA, to which the aqueous buffer solution prepared in **step (i)** is added and left stirring until homogenized and dissolved, and thereafter providing the desired amount of a lectin-binding molecule, the lectin-binding molecule being dextran having a molecular weight of 10-100 kDa, to which the said buffer solution containing said ConA is added and left stirring until homogenized and dissolved.

## Patentansprüche

1. Zusammensetzung zur Verwendung als aktives Fluid in einem kontinuierlichen Glukosesensor, umfassend:

   ein Lektin, wobei das Lektin Concanavalin A (ConA) ist;
   ein Lektin bindendes Molekül mit einem Molekulargewicht von 10-100 kDa, wobei das Lektin bindende Molekül Dextran ist;
   mindestens ein Chloridsalz eines zweiwertigen Metallions, ausgewählt aus $MgCl_2$, $CaCl_2$ und $MnCl_2$ oder einer Kombination davon,
   wobei die Zusammensetzung durch Folgendes gekennzeichnet ist:
   die Konzentration von ConA beträgt 1-1,5 mM auf Grundlage der Monomerkonzentration und die Konzentration von Dextran beträgt 1-1,5 mM, wobei der kontinuierliche Glukosesensor:

   - die Veränderungen der Konzentrationen von Glukose in Fluiden in vitro und in vivo durch Detektieren von Unterschieden des osmotischen Drucks misst und
   - Glukosekonzentrationen durch das Aufzeichnen des osmotischen Drucks zum Messen der Glukosekonzentrationen in vitro oder in vivo kontinuierlich nachverfolgt.

2. Zusammensetzung nach Anspruch 1, wobei das Molverhältnis von ConA zu Dextran 1:1 beträgt.

3. Zusammensetzung nach einem der vorhergehenden Ansprüche, wobei die Zusammensetzung durch Folgendes gekennzeichnet ist:

   das Dextran ist Dextran 40 kDa oder Dextran 70 kDa;
   eine wässrige Pufferlösung mit einem pH-Wert von 7,4-7,5, enthaltend Tris-Puffer 100 mM, 10

mM $MgCl_2$, 10 mM $CaCl_2$, 150 mM NaCl und 30 mM Glukose.

**4.** Verfahren zum Herstellen einer Zusammensetzung, die zur Verwendung als aktives Fluid in einem kontinuierlichen Glukosesensor nach den Ansprüchen 1-3 geeignet ist, umfassend die folgenden Schritte:

(i) Herstellen einer wässrigen Pufferlösung mit einem pH-Wert von 7,0 bis 7,8, umfassend mindestens ein Chloridsalz eines zweiwertigen Metalls, ausgewählt aus $MgCl_2$, $CaCl_2$ und $MnCl_2$ oder einer Kombination davon und optional Glukose,
(ii) entweder:

- (a) Bereitstellen der gewünschten Menge eines Lektin bindenden Moleküls, wobei das Lektin bindende Molekül Dextran mit einem Molekulargewicht von 10-100 kDa ist, zu dem die in Schritt (i) hergestellte wässrige Pufferlösung hinzugegeben wird und gerührt wird, bis sie homogenisiert und gelöst ist, und danach Bereitstellen der gewünschten Menge eines Lektins, wobei das Lektin ConA ist, zu dem die Pufferlösung, die das Dextran enthält, hinzugegeben wird und gerührt wird, bis sie homogenisiert und gelöst ist, oder
- (b) Bereitstellen der gewünschten Menge eines Lektins, wobei das Lektin ConA ist, zu dem die in Schritt (i) hergestellte wässrige Pufferlösung hinzugegeben und gerührt wird, bis sie homogenisiert und gelöst ist, und danach Bereitstellen der gewünschten Menge eines Lektin bindenden Moleküls, wobei das Lektin bindende Molekül Dextran mit einem Molekulargewicht von 10-100 kDa ist, zu dem die Pufferlösung, die das ConA enthält, hinzugegeben wird und gerührt wird, bis sie homogenisiert und gelöst ist.

## Revendications

**1.** Composition destinée à être utilisée comme fluide actif dans un capteur de glucose continu comprenant :

une lectine, la lectine étant la concanavaline A (ConA) ;
une molécule se liant à la lectine ayant un poids moléculaire de 10 à 100 kDa, la molécule se liant à la lectine étant le dextrane ;
au moins un sel chlorure d'un ion métallique divalent choisi parmi $MgCl_2$, $CaCl_2$ et $MnCl_2$ ou une combinaison de ceux-ci,
la composition étant **caractérisée par**
la concentration de ConA est de 1 à 1,5 mM sur la base de la concentration de monomère et la concentration de dextrane est de 1 à 1,5 mM, dans laquelle le capteur de glucose continu :

- mesure les changements dans la concentration de glucose dans des fluides in vitro ou in vivo en détectant des différences de pression osmotique, et
- suit des concentrations de glucose en continu à travers l'enregistrement de la pression osmotique, pour mesurer des concentrations de glucose in vitro ou in vivo.

**2.** Composition selon la revendication 1, dans laquelle le rapport molaire ConA au dextrane est de 1:1.

**3.** Composition selon l'une quelconque des revendications précédentes, dans laquelle la composition est **caractérisée par** :

le dextrane est du dextrane de 40 kDa ou du dextrane de 70 kDa ;
une solution tampon aqueuse à pH 7,4 à 7,5 contenant du tampon Tris à 100 mM, 10 mM de $MgCl_2$, 10 mM de $CaCl_2$, 150 mM de NaCl et 30 mM de glucose.

**4.** Procédé de préparation d'une composition appropriée destinée à être utilisée comme fluide actif dans un capteur de glucose continu selon les revendications 1 à 3, comprenant les étapes suivantes

(i) préparation d'une solution tampon aqueuse ayant un pH de 7,0 à 7,8, comprenant au moins un sel chlorure d'un métal divalent choisi parmi $MgCl_2$, $CaCl_2$ et $MnCl_2$ ou une combinaison de ceux-ci, et éventuellement du glucose,
(ii) soit :

- (a) fourniture de la quantité souhaitée d'une molécule se liant à la lectine, la molécule se liant à la lectine étant du dextrane ayant un poids moléculaire de 10 à 100 kDa, à laquelle la solution tampon aqueuse préparée à l'étape (i) est ajoutée et laissée sous agitation jusqu'à homogénéisation et dissolution, et fourniture ensuite de la quantité souhaitée d'une lectine, la lectine étant ConA à laquelle ladite solution tampon contenant ledit dextrane est ajoutée et laissée sous agitation jusqu'à homogénéisation et dissolution, soit
- (b) fourniture de la quantité souhaitée d'une lectine, la lectine étant ConA, à laquelle la solution tampon aqueuse préparée à l'étape (i) est ajoutée et laissée sous agitation jusqu'à homogénéisation et dissolution, et fourniture ensuite de la quantité souhaitée d'une molécule se liant à la lectine, la molécule se liant à la lectine étant du dextrane ayant un poids moléculaire de 10 à 100 kDa, à laquelle ladite solution tampon contenant ladite ConA est ajoutée et laissée sous agitation jusqu'à homogénéisation et dissolution.

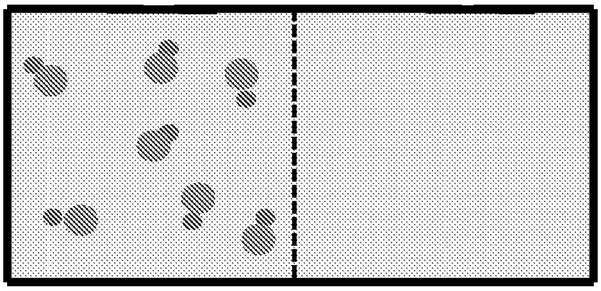

Fig. 1A

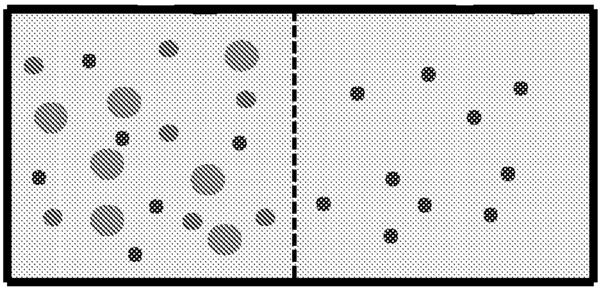

Fig. 1B

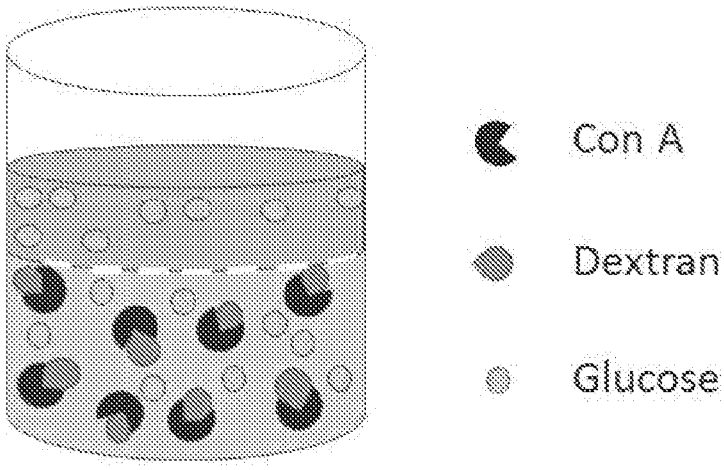


Fig. 1C

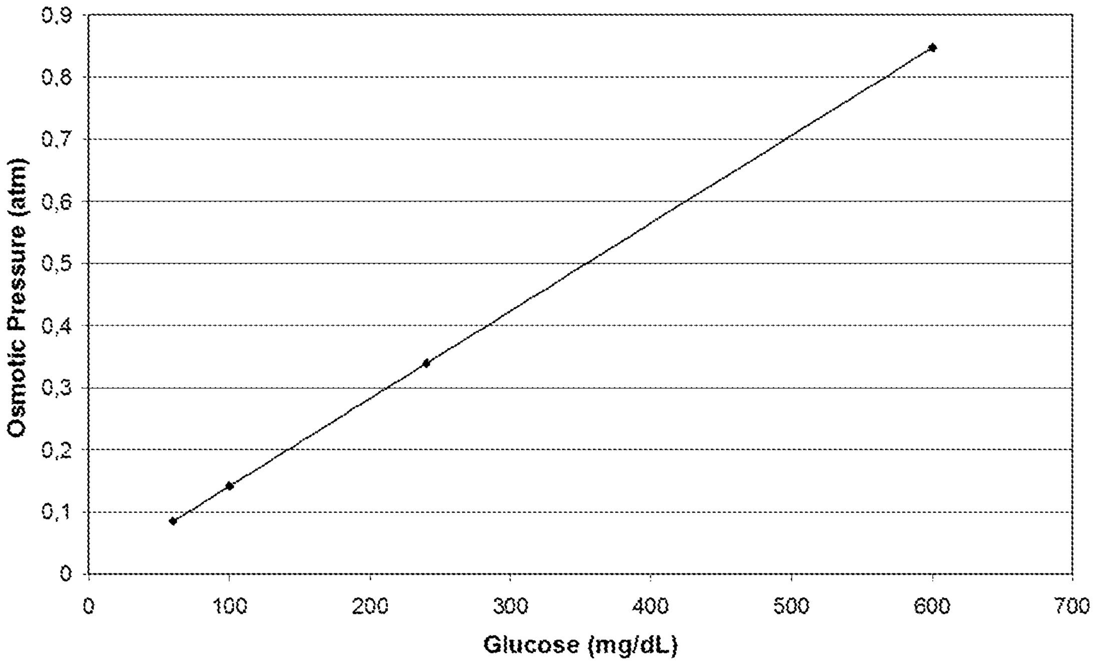
Osmotic Pressure (atm)
0,9
0,8
0,7
0,6
0,5
0,4
0,3
0,2
0,1
0
0
100
200
300
400
500
600
700
Glucose (mg/dL)

Fig. 1D

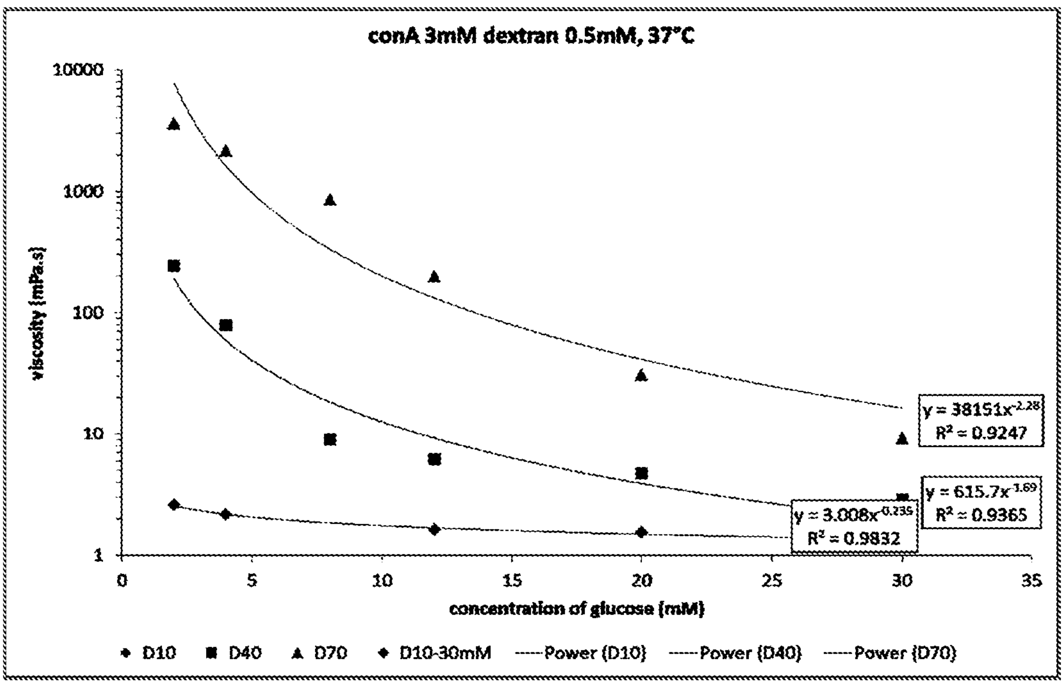
conA 3mM dextran 0.5mM, 37°C
viscosity (mPa.s)
10000
1000
100
10
1
0
5
10
15
20
25
30
35
concentration of glucose (mM)
y = 38151x^-2.28
R² = 0.9247
y = 615.7x^-1.69
R² = 0.9365
y = 3.008x^-0.235
R² = 0.9832
D10
D40
D70
D10-30mM
Power (D10)
Power (D40)
Power (D70)

Fig. 2

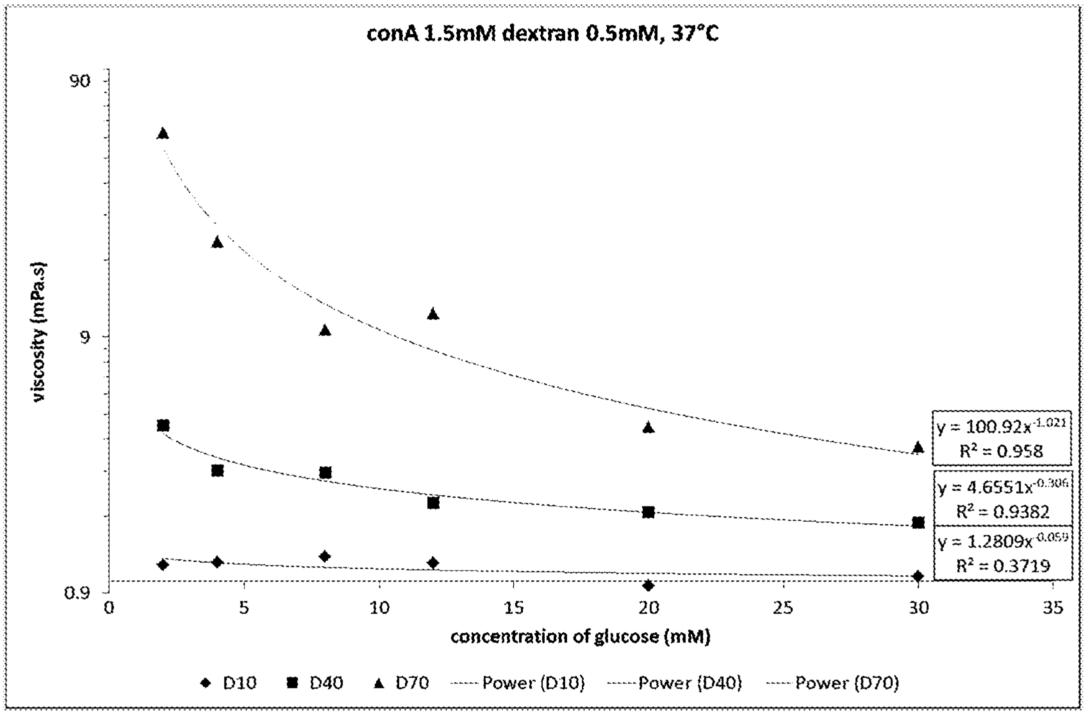
conA 1.5mM dextran 0.5mM, 37°C
90
9
0.9
viscosity (mPa.s)
0
5
10
15
20
25
30
35
concentration of glucose (mM)
y = 100.92x^-1.021
R² = 0.958
y = 4.6551x^-0.306
R² = 0.9382
y = 1.2809x^-0.059
R² = 0.3719
D10
D40
D70
Power (D10)
Power (D40)
Power (D70)

Fig. 3

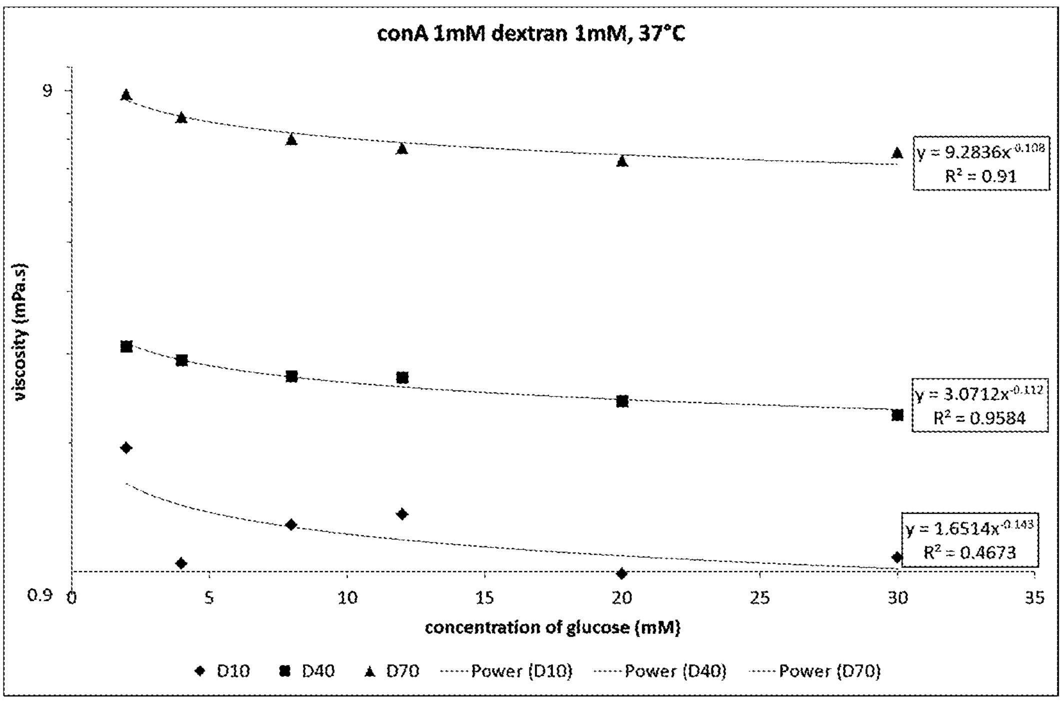
conA 1mM dextran 1mM, 37°C
9
0.9
viscosity (mPa.s)
0
5
10
15
20
25
30
35
concentration of glucose (mM)
y = 9.2836x^-0.108
R² = 0.91
y = 3.0712x^-0.112
R² = 0.9584
y = 1.6514x^-0.143
R² = 0.4673
D10
D40
D70
Power (D10)
Power (D40)
Power (D70)

Fig. 4

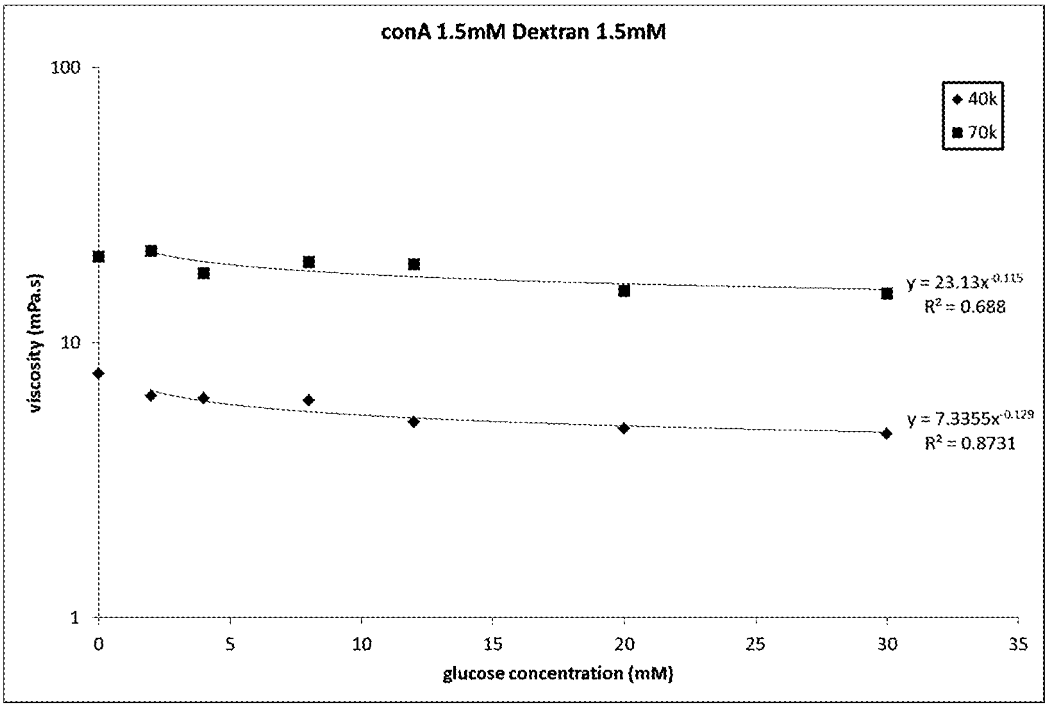
conA 1.5mM Dextran 1.5mM
40k
70k
y = 23.13x^-0.115
R² = 0.688
y = 7.3355x^-0.129
R² = 0.8731
viscosity (mPa.s)
glucose concentration (mM)
100
10
1
0
5
10
15
20
25
30
35

Fig. 5

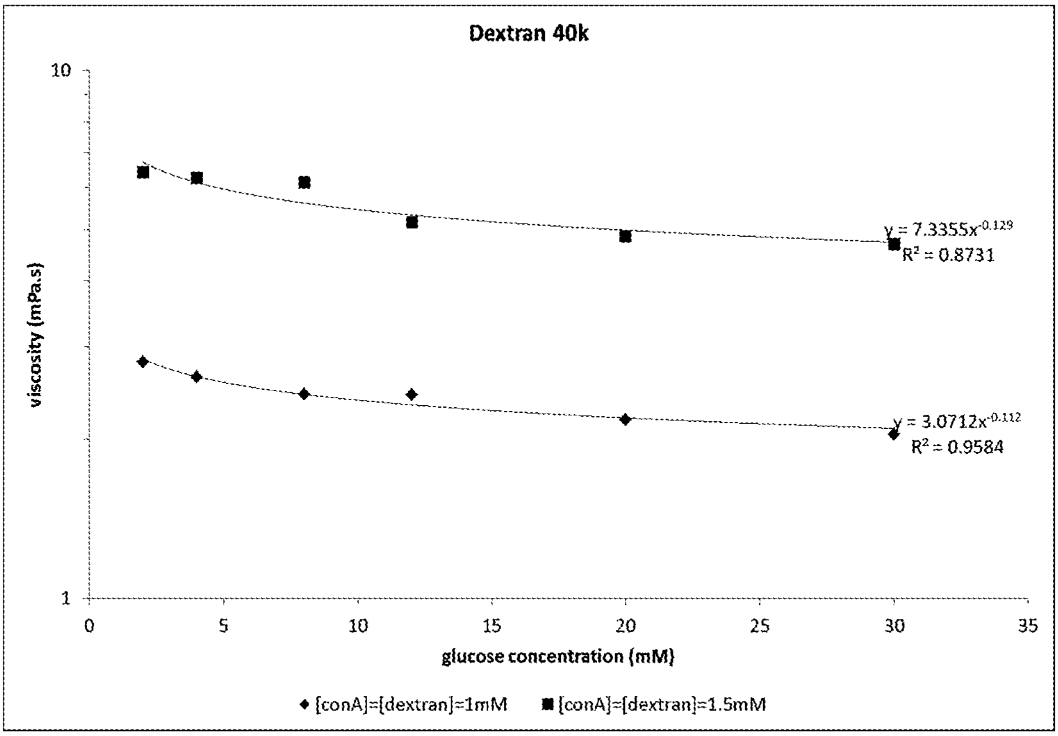
Dextran 40k
y = 7.3355x^-0.129
R² = 0.8731
y = 3.0712x^-0.112
R² = 0.9584
viscosity (mPa.s)
glucose concentration (mM)
10
1
0
5
10
15
20
25
30
35
[conA]=[dextran]=1mM
[conA]=[dextran]=1.5mM

Fig. 6

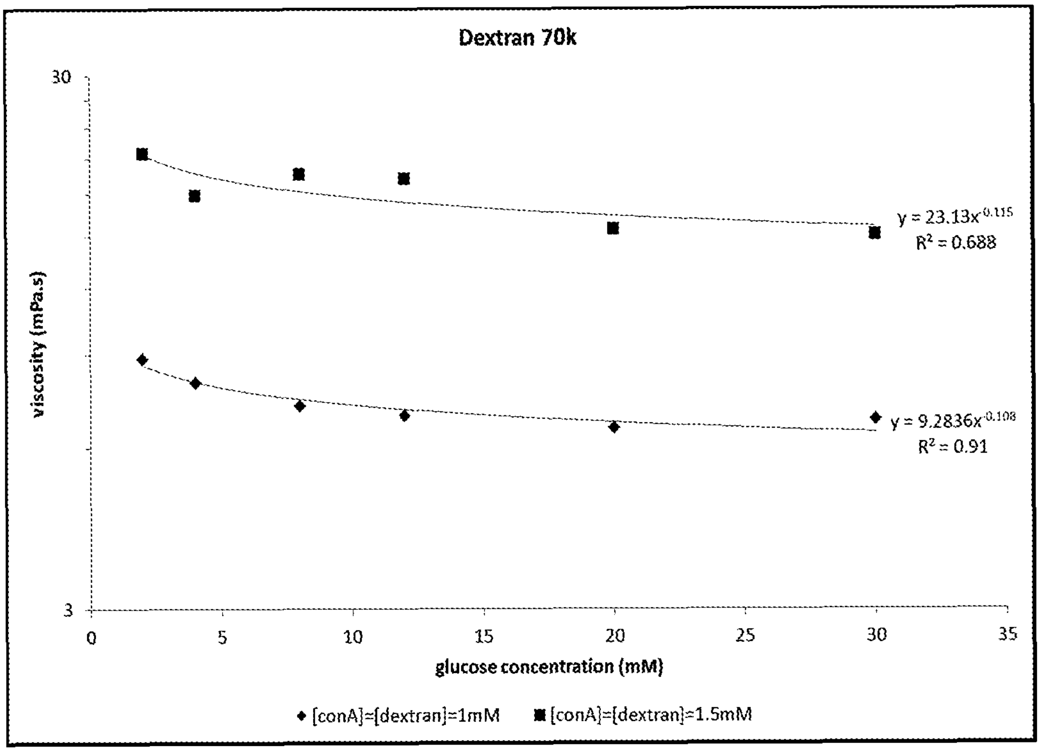
Dextran 70k
30
3
viscosity (mPa.s)
0
5
10
15
20
25
30
35
glucose concentration (mM)
y = 23.13x^-0.115
R² = 0.688
y = 9.2836x^-0.108
R² = 0.91
◆ [conA]=[dextran]=1mM
■ [conA]=[dextran]=1.5mM

Fig. 7

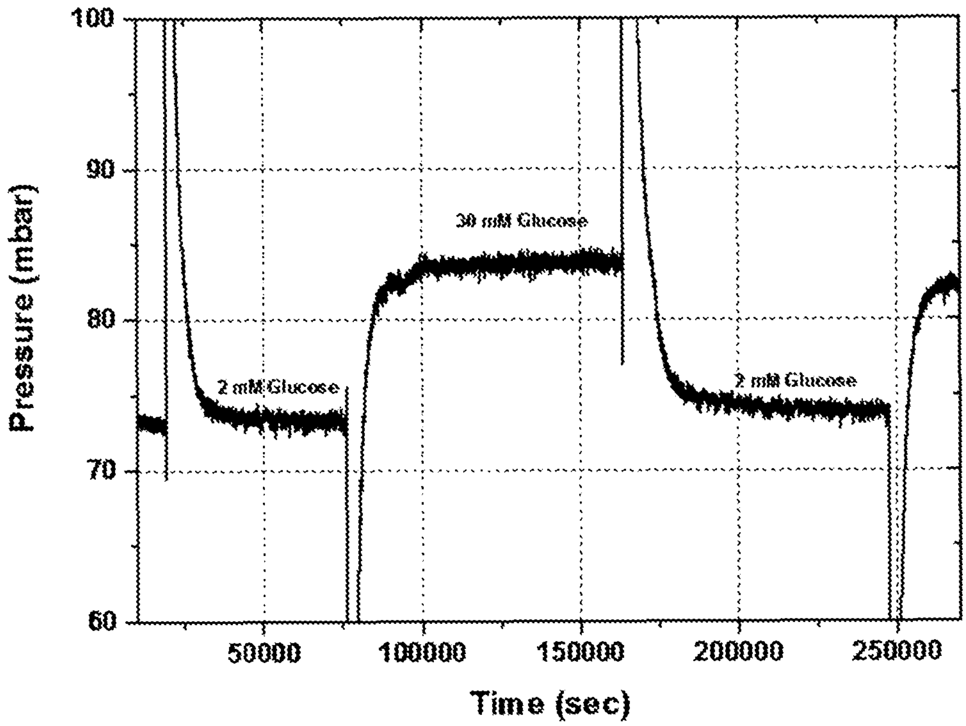
100
90
80
70
60
Pressure (mbar)
50000
100000
150000
200000
250000
Time (sec)
2 mM Glucose
30 mM Glucose
2 mM Glucose

Fig. 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 1631187 B1
- EP 2055226 A
- WO 2006061207 A
- WO 20047056311 A


### Non-patent literature cited in the description


- **OLGA KRUSHINITSKAYA,.** Osmotic sensor for blood glucose monitoring applications. Department of Micro- and Nanosystems Technology, August 2012
- The assessment of potentially interfering metabolites and dietary components in blood using an osmotic glucose sensor based on the concanavalin Adextran affinity assay. **OLGA KRUSHINITSKAYA et al.** BIOSENSORS AND BIOELECTRONICS. ELSEVIER BV, 09 July 2011, vol. 28, 195-203
- Rheological and diffusion properties of a dextran-con A polymer in the presence of Insulin and Magnesium. **ADAMS GARY G et al.** RHEOLOGICA ACTA. DIETRICH STEINKOPFF VERLAG, 08 November 2005, vol. 45, 611-620
- **RAMLJAK S et al.** *& Moderated Poster Discussion ADA-Supported Research CLINICAL THERAPEUTICS/NEW TECHNOLOGY- GLUCOSE MONITORING AND SENSING: In vitro proof of principle experiment with the osmotic pressurebased sencell implantable glucose sensor technology,* 10 June 2016
- **MEHVAR et al.** Dextran for trageted and sustained delivery of therapeutic and imaging agents. *Journal of Controlled release,* 2000, vol. 69, 1-25